(19) Europäisches Patentamt
European Patent Office
Office européen des brevets

(11) Numéro de publication : **0 244 328 B1**

# (12) FASCICULE DE BREVET EUROPEEN

(45) Date de publication du fascicule du brevet : **10.04.91 Bulletin 91/15**

(51) Int. Cl.[5] : **C07C 45/49**, C07C 47/52, C07D 213/00

(21) Numéro de dépôt : **87420098.3**

(22) Date de dépôt : **14.04.87**

## (54) Procédé de préparation d'aldéhydes aromatiques.

(30) Priorité : **28.04.86 FR 8606363**

(43) Date de publication de la demande : **04.11.87 Bulletin 87/45**

(45) Mention de la délivrance du brevet : **10.04.91 Bulletin 91/15**

(84) Etats contractants désignés : **AT BE CH DE ES GB GR IT LI LU NL SE**

(56) Documents cités :
EP-A- 0 109 606
EP-A- 0 165 881
EP-A- 0 206 957
US-A- 3 960 932
US-A- 4 605 749
JOURNAL OF THE AMERICAN CHEMICAL SOCIETY, vol. 96, no. 25, 11 décembre 1974, pages 7761-7764; A. SCHOENBERG et al.: "Palladium-catalyzed formylation of aryl, heterocyclic, and vinylic halides"

(73) Titulaire : **RHONE-POULENC CHIMIE**
**25, quai Paul Doumer**
**F-92408 Courbevoie Cédex (FR)**

(72) Inventeur : **Bluthe, Norbert**
**39, rue Clément Michut**
**F-69100 Villeurbanne (FR)**
Inventeur : **Perron, Robert**
**La Pecolière**
**F-69390 Charly (FR)**

(74) Mandataire : **Vignally, Noel et al**
**RHONE-POULENC INTERSERVICES Service Brevets Chimie Centre de Recherches de Saint-Fons B.P. 62**
**F-69192 Saint-Fons Cedex (FR)**

EP 0 244 328 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation d'aldéhydes aromatiques par hydrocarbonylation d'halogénures aromatiques en présence d'un métal noble, d'une base azotée tertiaire et d'un dérivé du phosphore.

Dans le brevet américain No. 3.960.932 on a décrit un procédé de préparation d'aldéhydes consistant à faire réagir un mélange gazeux d'hydrogène et de monoxyde de carbone avec un halogénure organique du groupe des halogénures aromatiques, vinyliques et hétérocycliques en présence d'une amine tertiaire et d'un catalyseur au palladium consistant en un complexe d'un dérivé du palladium divalent avec une phosphine (triphénylphosphine), un phosphite ou une arsine ou en l'association d'un sel de palladium divalent (acétate, chlorure) ou de palladium métallique finement divisé avec un agent complexant du groupe des phosphines, des phosphites ou des arsines. Dans ce dernier cas, le rapport nombre d'at-g de P/nombre d'at.g de Pd peut être compris entre 0,5 et 5. La réaction est conduite à une température de 75 à 175°C et sous une pression de 7 à 140 bar. En général la base azotée est utilisée en léger excès par rapport à la quantité théorique nécessaire à la neutralisation de l'hydracide sous produit de la réaction. Ce procédé convient tout spécialement bien à la préparation d'aldéhydes aromatiques par hydrocarbonylation des bromures correspondants. En dépit des bons rendements auxquels il conduit, ce procédé souffre d'un grave inconvénient qui réside dans des durées de réaction de l'ordre de 10 à 26 heures. Ces durées de réaction se traduisent par de faibles productivités de l'appareillage qui otent tout intérêt industriel au procédé.

Afin de pallier les inconvénients du procédé décrit dans le brevet américain 3.960.932, on a proposé dans la demande de brevet européen No. 0.109.606 de conduire l'hydrocarbonylation des halogénures aromatiques à des pressions de 20 à 400 bar, à une température de 80 à 250°C, en présence d'un catalyseur à base de métal noble, d'une base tertiaire azotée et d'une quantité importante d'une phosphine ou d'un phosphite. La quantité de dérivé phosphoré représente de 2 à $10^5$ fois la quantité molaire de catalyseur, et de préférence de 10 à 1000 fois. En permettant le recours à des températures de réaction élevées sans dégradation du catalyseur, ce procédé permet d'augmenter la vitesse de la réaction et par conséquent la productivité de l'appareillage. Toutefois l'augmentation de la vitesse de la réaction s'avère insuffisante et liée à la mise en oeuvre de températures élevées.

Dans le but d'élever encore la vitesse de la réaction, de préférence sans avoir à recourir à des températures élevées, la demanderesse a procédé à des études approfondies des paramètres de la réaction qui lui ont permis de constater que la concentration en base azotée tertiaire exerce une influence sur la vitesse de la réaction. On a constaté plus particulièrement que la vitesse de la réaction est d'ordre positif par rapport à la base azotée, ce qui signifie qu'à partir d'une certaine valeur de la concentration en base, la vitesse de la réaction est indépendante de cette concentration. Par la suite, an désignera par concentration en base azotée le nombre de mole de base par litre du mélange réactionnel base/halogénure aromatique/ et éventuellement solvant ou diluant organique.

Plus spécifiquement, la présente invention a pour objet un procédé d'obtention d'aldéhydes aromatiques par réaction d'un mélange hydrogène/oxyde de carbone avec un halogénure aromatique du groupe des bromure et iodure, en présence d'un catalyseur à base d'un métal noble, d'une base azotée tertiaire et éventuellement d'un agent complexant du métal noble pris dans le groupe des phosphines et des phosphites, caractérisé en ce que la concentration en base azotée exprimée en mole par litre de mélange réactionnel est maintenue à une valeur d'au moins 2 moles/litre pendant la durée de la réaction.

De préférence la concentration en base azotée est maintenue à au moins 2,5 moles par litre pendant la réaction.

Les diverses conditions de la réaction sont celles décrites les brevet et demande de brevets précités.

Ainsi, on peut utiliser à titre de catalyseurs pour la mise en oeuvre du procédé selon l'invention un métal noble finement divisé du groupe VIII de la classification périodique des éléments tels que le palladium, le rhodium ou l'iridium, ou leurs sels d'acides minéraux ou organiques ou leurs complexes avec des composés donneurs de doublets électroniques, en particulier leurs complexes avec les phosphines, les phosphites ou les arsines. Les dérivés du palladium conviennent tout particulièrement bien à la mise en oeuvre du procédé selon l'invention. Comme exemples spécifiques de dérivés du palladium, on peut citer les carboxylates (acétate, propionate, butyrate, benzoate) de palladium (II), le chlorure palladeux et les complexes du palladium de formules générales $Pdx_2[P(R)_3]_2$ ou $Pdx_2[P(OR)_3]_2$ lesquelles X représente un atome d'halogène (brome, chlore) ou un reste d'un acide minéral ou carboxylique et R un radical hydrocarboné. On peut citer en particulier le dichlorodi(triphénylphosphino)palladium (II) et le dibromodi(tritolylphosphino)palladium (II).

La quantité de catalyseur exprimée en atome-gramme de métal ou en mole de dérivé métallique par mole d'halogénure aromatique peut varier dans de larges limites. Ainsi elle peut être comrise entre $10^{-5}$ et $10^{-1}$ at.g ou moles par mole et de préférence entre $10^{-4}$ et $10^{-2}$ at.g ou moles par mole.

A titre de base azotée tertiaire, on peut faire appel à des amines de formule générale :

$$N-(R_1)_3$$

dans laquelle les radicaux $R_1$, identiques ou différents, représentent des restes hydrocarbonés comportant de 1 à 20 atomes de carbone tels que les radicaux alkyle, cycloalkyles ou aryle. De préférence les symboles $R_1$ représentent des radicaux alkyles comportant de 1 à 10 atomes de carbone ou des radicaux cycloalkyle ayant de 5 à 10 atomes de carbone. Comme exemple de telles bases on peut citer la triéthylamine, la tri-n-propylamine, la tri-n-butylamine, la méthyldibutylamine, la méthyldicyclohexylamine, l'éthyldiisopropylamine. On peut encore faire appel à des bases tertiaires, hétérocycliques telles que la pyridine et les picolines.

La quantité de base doit être suffisante à la fois pour neutraliser l'hydracide libéré par la réaction et pour que la concentration en base soit au moins de 2 moles par litre de mélange réactionnel pendant la durée de la réaction. Lorsque ces deux conditions sont satisfaites, il n'y a pas de limites supérieures critiques de la quantité de base qui peut être utilisée en large excès par rapport à la quantité théoriquement nécessaire à la neutralisation de l'hydracide formé. Pour maintenir la concentration en base au moins égale aux valeurs limites données précédemment pendant toute la durée de la réaction, la quantité de base doit être calculée pour qu'en fin de réaction la concentration en base soit au moins égale à ces valeurs. On peut également ajouter une quantité de base supplémentaire au fur et à mesure du déroulement de la réaction pour compenser celle consommée par la neutralisation de l'hydracide.

Les phosphines et les phosphites qui conviennent au déroulement de la réaction sont ceux cités dans le brevet américain 3.960.932 ou dans la demande européenne 0.109.606 dont le contenu est inclu par référence. On peut citer comme exemples non limitatifs de ces composés : la triphénylphosphine, le triphénylphosphite, la diéthylphénylphosphine, le diéthylphénylphosphite, la tritolylphosphine, le tritolylphosphite, la trinaphtylphosphine, le trinaphtylphosphite, la diphénylméthylphosphine, le diphénylmétylphosphite, la diphénylbutylphosphite, le diphénylbutylphosphite, la tris-(p-méthoxycarbonylphényl)-phosphine, le tris-(p-méthoxycarbonylphényl)-phosphite, la tris-(p-cyanophényl)-phosphine, le tris-(cyanophényl)-phosphite, le triéthylphosphite, la tributylphosphine, le tributylphosphite.

La présence d'un agent complexant phosphoré libre dans le milieu réactionnel dépend de la nature du catalyseur et/ou des conditions de la réaction. Lorsque le catalyseur est constitué par un complexe d'un métal noble avec une phosphine ou un phosphite, la présence de ces derniers à l'état libre n'est pas indispensable. Toutefois elle s'avère avantageuse lorque la réaction est conduite à température élevée, par exemple à température supérieure à 150°C. Lorsqu'on met en oeuvre un métal noble à l'état métallique ou un dérivé non complexé par une phosphine ou un phosphite, tel que les carboxylates de métaux nobles par exemple, il est nécessaire d'opérer en présenoe du composé phosphoré. Lorsqu'on conduit la réction en présence d'un phosphite ou d'une phosphine la quantité en est choisie de façon à ce que le rapport du nombre d'atome-gramme de phosphore (P) au nombre d'atome-gramme de métal (M) soit au moins égal à 2. Le rapport P/M peut prendre des valeurs aussi élevées que 10.000. Un rapport P/M compris entre 5 et 1000 est en général convenable.

Le procédé selon l'invention convient tout spécialement bien à la préparation d'aldéhydes aromatiques de formule générale :

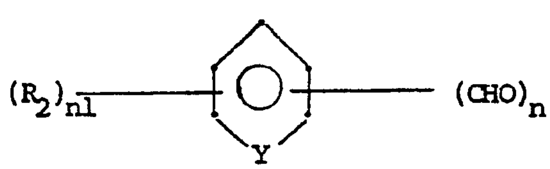

(I)

dans laquelle :

- n est 1 ou 2 ;
- $n_1$ est un nombre entier de 1 à 4 ;
- $R_2$ représente : un atome d'hydrogène, de fluor ou de chlore ; un radical alkyle éventuellement substitué par un ou plusieurs atomes de chlore et/ou de fluor, cycloalkyle, aryle, alkoxy, cycloalkoxy, aryloxy, alkoxycarbonyle, cycloalkoxycarbonyle, aryloxycarbonyle, alkyl-, cycloalkyl- ou arylcarbonyloxy, éventuellement substitués par un ou plusieurs atomes de fluor et/ou de chlore ; un groupe nitrile ou, lorsque $n_1$ est égal à 2, deux radicaux $R_2$ portés par des atomes de carbone vicinaux forment avec ces derniers un cycle hydrocarboné ou un hétérocycle.

Lorsque $n_1$ est supérieur à 1, les différents substituants $R_2$ peuvent être identiques ou différents.
- Y représente un reste divalent –CH– ou un atome d'azote.

Dans la formule (I) $R_2$ représente plus particulièrement :

- des radicaux alkyles linéaires ou ramifiés comportant de 1 à 20 atomes de carbone tels que les radicaux

méthyle, éthyle, propyles, butyles, pentyles, éthyl-2 hexyle, trifluorométhyle, difluorochlorométhyle, trichlorométhyle, décyles. De préférence $R_2$ représente un radical alkyle inférieur (ayant de 1 à 4 atomes de carbone).

• des radicaux cycloalkyles comportant de 5 à 10 atomes de carbone : cyclopentyle, cyclohexyle, cyclooctyle.

• des radicaux phényles éventuellement substitués par un ou plusieurs radicaux alkyles inférieurs ou alkoxy inférieurs, tels que phényle, xylyles, toluyles, méthoxyphényles, éthoxyphényles.

• des radicaux alkoxy comportant de 1 à 20 atomes de carbone, de préférence de 1 à 10, tels que les radicaux méthoxy, éthoxy, isopropyloxy, butoxy, trifluorométhoxy, difluorochlorométhoxy, trichlorométhoxy.

• des radicaux alkoxycarbonyles comportant de 1 à 10 atomes de carbone dans le reste alkoxy, de préférence des radicaux alkoxycarbonyles inférieurs tels que méthoxy-, éthoxy-, isopropyloxy-, butyloxycarbonyle.

• des radicaux cycloalkoxycarbonyles comportant de 5 à 10 atomes de de carbone, tels que les radicaux cylclopentyloxycarbonyle, cyclohexyloxycarbonyle.

• des radicaux phényloxycarbonyle, tolyxycarbonyle.

• des radicaux alkylcarbonyloxy comportant de 1 à 10 atomes de carbone tels que acétoxy, propionyloxy, butyroyloxy.

• des radicaux cycloalkylcarbonyloxy ayant de 5 à 10 atomes de carbone tels que cyclopentanoyloxy, cyclohexanoyloxy.

• des radicaux benzoyloxy, méthylbenzoyloxy, diméthylbenzoyloxy.

• lorsque deux radicaux $R_2$ forment un cycle avec les atomes de carbone vicinaux qui les portent, ce cycle peut être plus particulièrement un cycle benzénique éventuellement substitués par des radicaux alkyle ou alkoxy inférieurs, ou un cycle méthylènedioxy (dioxa-1,3 cyclopentane).

Comme exemples spécifiques d'aldéhydes de formule (I) qui peuvent être préparés par le procédé de l'invention on peut citer : le benzaldéhyde, les tolualdéhydes, les anisaldéhydes, la vanilline, le triméthoxybenzaldéhyde, le méthylènedioxy-3,4 benzaldéhyde (pipéronal), le téréphtalaldéhyde, les o-, m- ou p-trifluorométhoxybenzaldéhydes, les o-, m- ou p-trichlorométhoxybenzaldéhydes, les o-, m- ou p-difluorochlorométhoxybenzaldéhydes, les naphtylaldéhydes, les formylpyridines.

Comme halogénures aromatiques constituant les produits de départ du procédé selon l'invention, on peut faire appel à des produits de formule générale :

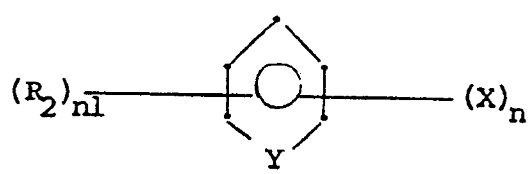

(II)

dans laquelle n, $n_1$, $R_2$ et Y ont la signification donnée ci-avant et X représente un atome de brome ou d'iode. A titre d'exemples non limitatifs de tels composés, on peut citer : le bromobenzène, les o-, m- et p-bromotoluènes, le diméthyl-2,3 bromobenzène, le diméthyl-2,4 bromobenzène, les o-, m- et p-trifluorobenzènes, les o-, m- et p-fluoroiodobenzènes, le difluoro-2, 3 bromobenzène, les o-, m- et p-trifluorométhylbromobenzènes, les o-, m- et p-trifluorométhyliodobenzènes, les trifluorométhoxybromobenzènes, les trifluorométhoxyiodobenzènes, les o-, m- et p-difluorochlorométhylbenzènes, les difluorochlorométhoxybromobenzènes, les bromobenzonitriles, les dibromobenzènes, le bromo-1 naphtalène, la bromo-2 pyridine, la bromo-4 pyridine, le p-bromodiphényléther, les bromobenzoates de méthyle, la p-bromoanisole, l'orthobromoanisole, la p-bromophénétole, le diméthoxy-3,4 bromobenzène, le triméthoxy-3,4,5 bromobenzène, le bromo-3 méthylènedioxybenzène.

La température à laquelle est mis en oeuvre le procédé selon l'invention peut varier dans de larges limites. En général une température prenant toute valeur de l'intervalle de 50 à 250°C convient bien. De préférence la température est comprise entre 5 et 200°C.

La pression totale du mélange hydrogène/monoxyde de carbone peut prendre toute valeur dans un intervalle de 1 à 400 bar et de préférence de 10 à 250 bar. Le rapport volumique $H_2/CO$ peut varier dans de larges limites. En général il peut être compris entre 0,1 et 10, et de préférence entre 0,2 et 5.

Le procédé selon la présente invention est conduit en phase liquide. On peut faire appel le cas échéant à un solvant inerte dans les conditions de la réaction. A cet effet on peut utiliser des hydrocarbures aliphatiques ou cycloaliphatiques saturés (hexane, cyclohexane) ou aromatiques : benzène, toluène, xylène ; des esters tels que le benzoate de méthyle, le téréphtalate de méthyle, l'adipate de méthyle, le phtalate de dibutyle, des

esters ou des éthers de polyols tels que le diacétate de tétraéthylèneglycol, des éthers cycliques (tétrahydrofuranne, dioxane).

D'un point de vue pratique le procédé selon l'invention est mis en oeuvre de façon simple par introduction dans un autoclave inerte de l'halogénure d'aryle, de la base azotée, du catalyseur et le cas échéant du dérivé phosphoré et d'un solvant puis alimentation dans l'autoclave fermé d'une pression adéquate d'un mélange $CO/H_2$. Le contenu de l'autoclave est ensuite porté sous agitation à la température convenable jusqu'à ce que l'absorption de gaz cesse. La pression dans l'autoclave peut être maintenue constante pendant la durée de la réaction en le reliant à une réserve de mélange gazeux à la pression choisie. En fin de réaction le contenu de l'autoclave est refroidi, l'autoclave dégazé et la masse réactionnelle est filtrée pour séparer l'halohydrate de la base azotée. Le filtrat est ensuite distillé pour séparer les constituants organiques du milieu réactionnel. Le résidu de distillation contenant le catalyseur peut être recyclé pour une nouvelle opération.

Le procédé selon l'invention peut être mis en oeuvre en continu ou en discontinu.

Les exemples suivants illustrent l'invention et montrent comment elle peut être mise en pratique.

## EXEMPLE 1

Dans un autoclave en acier inoxydable de 125 ml purgé préalablement à l'argon on charge :

- 5,025 g (25 mmoles) de bromométhylènedioxybenzène (environ 3 ml)
- 10,1 g (100 mmoles) de triéthylamine (environ 13, 9 ml)
- 224 mg de diacétate de palladium
- 1,31 g de triphényphosphine
- 20 ml de phtalate de dibutyle.

Le réacteur est fermé, agité par un système de va et vient, porté à 135°C et relié à une réserve d'un mélange $CO/H_2$ à un volume par volume. La pression dans l'autoclave est maintenue à 30 bar. La chute de pression dans la réserve permet de suivre le déroulement de la réaction. On constate que l'absorption de gaz cesse après 1 h 40 mn dans ces conditions. L'autoclave est refroidi puis dégazé et la masse réactionnelle est analysée par chromatographie en phase gazeuse. On constate qu'il s'est formé 3,11 g de pipéronal, ce qui représente un rendement par rapport au bromométhylènedioxybenzène chargé (rendement réel ou RR) de 82%. La concentration initiale de la base a été de 2,7 et la concentration finale de 2,46.

La vitesse initiale de la réaction exprimée en mmole de gaz absorbé par heure est de 80 mmoles $h^{-1}$.

## EXEMPLE 2

On a répété l'exemple 1 en faisant varier la concentration initiale de base dans le milieu. On a obtenu les résultats consignés dans le tableau suivant :

| EX. | Triéthyl-amine mmoles | Concentration en triéthylamine | | DURÉE (h) | RR % | Vitesse initiale mmol $h^{-1}$ |
|---|---|---|---|---|---|---|
| | | Initiale moles/l | Finale moles/l | | | |
| 2 | 150 | 3,42 | 3,34 | 1 h 40 | 80 | 77 |
| ESSAIS | | | | | | |
| A | 30 | 1,1 | 0,24 | 7 h 30 | - | 16 |
| B | 55 | 1,8 | 1,24 | 2 h | 82 | 34 |

EXEMPLES 3 à 4

On opère comme aux exemples 1, 2 et à l'essai B mais en diminuant la quantité de catalyseur de moitié (0,5 mmol de diacétate de palladium). On a obtenu les résultats suivants :

| EX. | Triéthylamine mmoles | Triéthylamine Concentration mole $l^{-1}$ initiale | Triéthylamine Concentration mole $l^{-1}$ finale | Vitesse intiale mmoles $h^{-1}$ |
|---|---|---|---|---|
| 3 | 100 | 2,7 | > 2,46 | 29 |
| 4 | 150 | 3,42 | > 3,34 | 27 |
| ESSAI C | 55 | 1,8 | 1,24 | 15 |

On constate que quelle que soit la quantité de catalyseur mise en oeuvre, la concentration en base exerce la même influence sur la vitesse de la réaction.

**Revendications**

1. Procédé d'obtention d'aldéhydes aromatiques par réaction d'un mélange hydrogène/oxyde de carbone avec un halogénure aromatique du groupe des bromure et iodure, en présence d'un catalyseur à base d'un métal noble, d'une base azotée tertiaire et éventuellement d'un agent complexant du métal noble pris dans le groupe des phosphines et des phosphites, caractérisé en ce que la concentration en base azotée exprimée en mole par litre de mélange réactionnel est maintenue à une valeur d'au moins 2 moles/litre pendant la durée de la réaction.

2. Procédé selon la revendication 1, caractérisé en ce que la concentration en base azotée est au moins égale à 2,5 moles par litre.

3. Procédé selon l'une quelconque des revendications 1 à 2, caractérisé en ce que l'on prépare des aldéhydes aromatiques de formule générale :

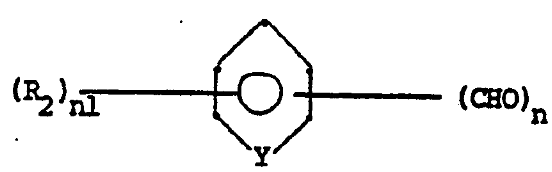

(I)

dans laquelle :

– n est 1 ou 2 ;

– $n_1$ est un nombre entier de 1 à 4 ;

– $R_2$ représente un atome d'hydrogène, de fluor ou de chlore, un radical alkyle éventuellement substitué par un ou plusieurs atomes de chlore et/ou de fluor, cycloalkyle, aryle, alkoxy, cycloalkoxy, aryloxy, alkoxycarbonyle, cycloalkoxycarbonyle, aryloxycarbonyle, alkyl-, cycloalkyl- ou arylcarbonyloxy, éventuellement substitués par un ou plusieurs atomes de fluor et/ou de chlore, nitrile ou, lorsque $n_1$ est égal à 2, deux radicaux $R_2$ portés par des atomes de carbone vicinaux forment avec ces derniers un cycle hydrocarboné ou un hétérocycle ;

– Y représente un reste divalent –CH– ou un atome d'azote ; à partir d'halogénures d'aryle de formule générale :

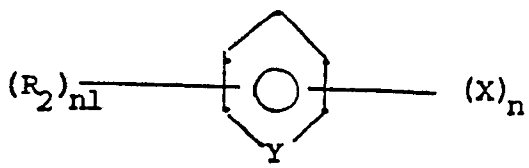

(II)

dans laquelle n, $n_1$, $R_2$ et Y ont la signification donnée ci-avant et X représente un atome de brome ou d'iode.

4. Procédé selon l'une quelconque des revendications 1 à 3, caractérisé en ce que la base azotée tertiaire est une amine.

5. Procédé selon l'une quelconque des revendication 1 à 4, caractérisé en ce que la température est comprise dans un intervalle de 50 à 250°C.

6. Procédé selon l'une quelconque des revendications 1 à 5, caractérisé en ce que la pression totale du mélange hydrogène/monoxyde de carbone est comprise dans un intervalle de 1 à 400 bar.

7. Procédé selon l'une quelconque des revendications 1 à 6, caractérisé en ce que le rapport en volume de $H_2/CO$ est compris dans un intervalle de 0,1 à 10.

8. Procédé selon l'une quelconque des revendications 1 à 7, caractérisé en ce que le catalyseur est constitué par du palladium métallique ou un dérivé du palladium.

9. Procédé selon l'une quelconque des revendications 1 à 8, caractérisé en ce que la quantité de palladium exprimée en atome-gramme de métal noble ou en mole de dérivé métallique par mole d'halogénure aromatique est comprise dans un intervalle de $10^{-5}$ à $10^{-1}$.

10. Procédé selon l'une quelconque des revendications 1 à 9, caractérisé en ce que la quantité de dérivé phosphoré est telle que le rapport du nombre des atome-gramme de phosphore au nombre des atome-gramme de métal soit compris dans un intervalle de 2 à 10.000.

## Ansprüche

1. Verfahren zur Herstellung von aromatischen Aldehyden durch Umsetzen eines Gemisches aus Wasserstoff und Kohlenoxid mit einem aromatischen Halogenid aus der Gruppe der Bromide und Iodide in Gegenwart eines Katalysators auf der Basis eines Edelmetalls, einer tertiären Stickstoffbase und gegebenenfalls eines Komplex-bildenden Mittels für das Edelmetall aus der Gruppe der Phosphine und Phosphite, dadurch gekennzeichnet, daß die Konzentration an Stickstoffbase ausgedrückt in mol/l Reaktionsgemisch während der Reaktionsdauer bei einem Wert von mindestens 2 mol/l gehalten wird.

2. Verfahren nach Anspruch 1, dadurch gekennzeichnet, daß die Konzentration an Stickstoffbase mindestens gleich 2,5 mol/l ist.

3. Verfahren nach einem der Ansprüche 1 bis 2, dadurch gekennzeichnet, daß man aromatische Aldehyde der allgemeinen Formel

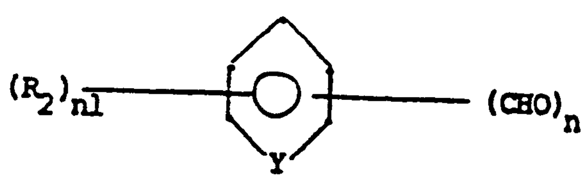

(I)

in der

– n 1 oder 2 ist ;

– $n_1$ eine ganze Zahl von 1 bis 4 ist ;

– $R_2$ für ein Wasserstoff-, Fluor- oder Chloratom, eine Alkylgruppe, gegebenenfalls substituiert mit einem oder mehreren Chlor- und/oder Fluoratomen, eine Cycloalkyl-, Aryl-, Alkoxy-, Cycloalkoxy-, Aryloxy-, Alkoxycarbonyl-, Cycloalkoxycarbonyl-, Aryloxycarbonyl-, Alkyl-, Cycloalkyl- oder Arylcarbonyloxygruppe, gegebenenfalls substituiert mit einem oder mehreren Fluor und/oder Chloratomen, eine Nitrilgruppe steht oder wenn $n_1$ gleich 2 ist, zwei Gruppen $R_2$, die an benachbarte Kohlenstoffatome gebunden sind, mit diesen letzteren einen Kohlenwasserstoffring oder einen Heterocyclus bilden ;

– Y eine zweiwertiqe Gruppe –CH– oder ein Stickstoffatom bedeutet ; ausgehend von Arylhalogeniden der allgemeinen Formel

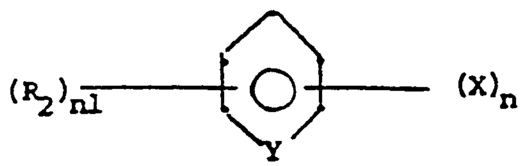

(II)

in der n, $n_1$, $R_2$ und Y die vorstehend angegebene Bedeutung haben und X für ein Brom- oder Iodatom steht, herstellt.

4. Verfahren nach einem der Ansprüche 1 bis 3, dadurch gekennzeichnet, daß die tertiäre Stickstoffbase ein Amin ist.

5. Verfahren nach einem der Ansprüche 1 bis 4, dadurch gekennzeichnet, daß die Temperatur im Bereich von 50 bis 250°C liegt.

6. Verfahren nach einem der Ansprüche 1 bis 5, dadurch gekennzeichnet, daß der Gesamtdruck des Gemisches aus Wasserstoff und Kohlenmonoxid im Bereich von 1 bis 400 bar liegt.

7. Verfahren nach einem der Ansprüche 1 bis 6, dadurch gekennzeichnet, daß das Volumenverhältnis von $H_2/CO$ in einem Bereich von 0,1 bis 10 liegt.

8. Verfahren nach einem der Ansprüche 1 bis 7, dadurch gekennzeichnet, daß der Katalysator aus metallischem Palladium oder einer Palladiumverbindung besteht.

9. Verfahren nach einem der Ansprüche 1 bis 8, dadurch gekennzeichnet, daß die Menge an Palladium ausgedrückt in Gramm-Atom Edelmetall oder in mol Metallverbindung je mol aromatisches Halogenid im Bereich von $10^{-5}$ bis $10^{-1}$ liegt.

10. Verfahren nach einem der Ansprüche 1 bis 9, dadurch gekennzeichnet, daß die Menge an Phosphorverbindung so beschaffen ist, daß das Verhältnis von Anzahl Gramm-Atom Phosphor zu Anzahl Gramm-Atom Metall im Bereich von 2 bis 10 000 liegt.

## Claims

1. Process for obtaining aromatic aldehydes by reacting a hydrogen/carbon monoxide mixture with an aromatic halide of the bromide and iodide group, in the presence of a noble metal-based catalyst, a tertiary nitrogenous base and, if required, a noble metal complexing agent chosen from the group consisting of phosphines and phosphites, characterized in that the concentration of nitrogenous base expressed in moles per litre of reaction mixture is maintained at a value of at least 2 moles/litre throughout the reaction period.

2. Process according to Claim 1, characterized in that the concentration of nitrogenous base is at least equal to 2.5 moles per litre.

3. Process according to either of Claims 1 and 2, characterized in that aromatic aldehydes of the following general formula :

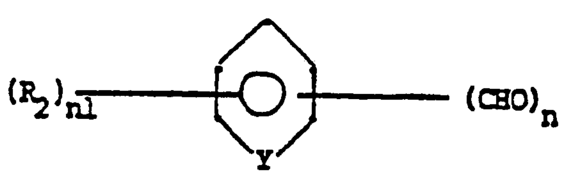

(I)

in which :

n is 1 or 2 ;

$n_1$ is an integer from 1 to 4 ;

$R_2$ represents a hydrogen, fluorine or chlorine atom, an alkyl radical which may be substituted, if required, with one or more chlorine and/or fluorine atoms, a cycloalkyl, aryl, alkoxy, cycloalkoxy, aryloxy, alkoxycarbonyl, cycloalkoxycarbonyl, aryloxycarbonyl, alkyl-, cycloalkyl- or arylcarbonyloxy radical, which may be substituted, if required, with one or more fluorine and/or chlorine atoms, a nitrile group or, when n is equal to 2, two $R_2$ radicals carried by neighbouring carbon atoms form a hydrocarbon ring or a heterocycle with the latter ; and

Y represents a divalent residue –CH– or a nitrogen atom ; are prepared from aryl halides of general formula:

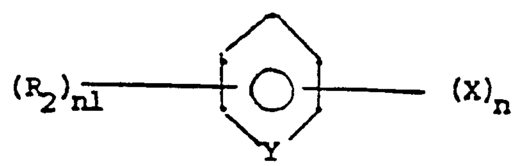

(II)

in which n, $n_1$, $R_2$ and Y have the meaning given above and X represents a bromine or iodine atom.

4. Process according to any one of Claims 1 to 3, characterized in that the tertiary nitrogenous base is an amine.

5. Process according to any one of Claims 1 to 4, characterized in that the temperature is within the range from 50 to 250°C.

6. Process according to any one of Claims 1 to 5, characterized in that the total pressure of the hydrogen/carbon monoxide mixture is within the range from 1 to 400 bar.

7. Process according to any one of Claims 1 to 6, characterized in that the $H_2/CO$ volume ratio is within the range from 0.1 to 10.

8. Process according to any one of Claims 1 to 7, characterized in that the catalyst consists of palladium metal or a palladium derivative.

9. Process according to any one of Claims 1 to 8, characterized in that the quantity of palladium expressed as gram-atoms of noble metal or as moles of metal derivative per mole of aromatic halide is within the range from $10^{-5}$ to $10^{-1}$.

10. Process according to any one of Claims 1 to 9, characterized in that the quantity of phosphorus derivative is such that the ratio of the number of gram-atoms of phosphorus to the number of gram-atoms of metal is within the range from 2 to 10,000.